# EUROPEAN PATENT SPECIFICATION

(11) **EP 4 030 989 B1**
(45) Date of publication and mention of the grant of the patent: **08.11.2023**
(21) Application number: 20771857.8
(22) Date of filing: 15.09.2020
(51) Int. Cl.: A61B 5/00

(54) **SYSTEM AND METHOD FOR BRAIN TISSUE ANALYSIS**
SYSTEM UND VERFAHREN ZUR ANALYSE VON GEHIRNGEWEBE
SYSTÈME ET PROCÉDÉ D'ANALYSE DE TISSU CÉRÉBRAL

(30) Priority: 16.09.2019 IT 201900016424
(43) Date of publication of application: 27.07.2022
(73) Proprietor: Consiglio Nazionale Delle Ricerche - CNR, 00185 Roma (IT); Universita' Degli Studi G. D Annunzio Chieti - Pescara, 66100 Chieti (IT)
(72) Inventor: LOMBARDO, Salvatore, Antonino, 00185 Roma RM (IT); MAIRA, Giovanni, Aldo, 00185 Roma RM (IT); LIBERTINO, Sebania, 00185 Roma RM (IT); MERLA, Arcangelo, 66100 Chieti CH (IT); CHIARELLI, Antonio, Maria, 66100 Chieti CH (IT)
(74) Representative: Perani & Partners S.p.A.
(86) International application number: PCT/EP2020/075774
(87) International publication number: WO 2021/052966

(56) References cited:
- DOMINIK WYSER ET AL: "Wearable and modular functional near-infrared spectroscopy instrument with multidistance measurements at four wavelengths", NEUROPHOTONICS, vol. 4, no. 04, 18 August 2017 (2017-08-18), page 1, XP055618655, 1000 20th St. Bellingham WA 98225-6705 USA ISSN: 2329-423X, DOI: 10.1117/1.NPh.4.4.041413
- ANTONIO M. CHIARELLI ET AL: "Characterization of a fiber-less, multichannel optical probe for continuous wave functional near-infrared spectroscopy based on silicon photomultipliers detectors: in-vivo assessment of primary sensorimotor response", NEUROPHOTONICS, vol. 4, no. 03, 27 September 2017 (2017-09-27), page 1, XP055693330, 1000 20th St. Bellingham WA 98225-6705 USA ISSN: 2329-423X, DOI: 10.1117/1.NPh.4.3.035002
- SANFILIPPO D ET AL: "Design and development of a fNIRS system prototype based on SiPM detectors", PROCEEDINGS OF SPIE/ IS & T,, vol. 8990, 8 March 2014 (2014-03-08), pages 899016-899016, XP060036004, DOI: 10.1117/12.2037559 ISBN: 978-1-62841-730-2

## Description

### Technical field

The present invention is related to a system and a corresponding method for brain tissue analysis.

The system and method are useful for functional Near-Infrared Spectroscopy (fNIRS) applications and/or for Diffuse Optical Tomography (DOT) applications.

The invention has been developed under the project H2020 - EU.2.1.1.7. ECSEL "ASTONISH, Advancing Smart Optical Imaging and Sensing for Health", 692470.

Link:
https://www.imm.cnr.it/proiects/astonish-advancing-smart-optical-imaging-and-sensing-health
https://cordis.europa.eu/proiect/rcn/203399/factsheet/itEuropean

### State of the art

Functional Near-Infrared Spectroscopy (fNIRS) is known in the art as a technique for brain tissue analysis. In particular, fNIRS is a non-invasive diagnostic technique used for monitoring the level of oxygenation of the human brain cortex. The fNIRS operates thanks to the relative transparency of human tissues in the near infrared (NIR) spectrum. In this range, the light absorption is mainly due to the oxygenated and the deoxygenated hemoglobin (O₂Hb and HHb respectively) in the blood. In particular, fNIRS is based on the detection of NIR backscattered photons after they have passed through human brain tissues. These backscattered photons carry information regarding the dynamical change of O₂Hb and HHb concentration in blood, providing relevant markers of hemodynamic and metabolic changes associated with neural activity in the brain.

Generally, a system for fNIRS comprises a plurality of optodes, namely a plurality of optical and electrical associations between an optical element and an optical sensor. Each optical element comprises a couple of emitting sources each one configured to emit light at a given mean wavelength with full width half maximum at least less than 50% of the mean when the optical element is supplied by an electrical power. At the same time, each optical sensor is configured to change from a rest state to an excited state when excited by light. In addition, the system comprises a source of electrical power configured to supply the optical element with a given value of electrical power, a source of electric voltage configured to supply each optical sensor with a given value of electric voltage and a measuring unit configured to measure the value of the excited state of the optical sensor.

In order to detect the neural activity of the brain, the optical elements and the optical sensors have to be disposed on the head of the patient under test according to a given geometrical pattern. In fact, the physical distance between each optical element and each sensor corresponds to a particular region of analysis inside the brain, approximately located at a certain depth. After activating each optode by supplying its optical element with a given value of electrical power and by supplying its optical sensor with the given value of electric voltage, light, emitted by the emitting sources of the optical element, can diffuse through the brain tissue. The portion of the light not absorbed by the brain tissue, and backward scattered towards the optical sensor, namely a portion of the backscattered light, excites the optical sensor of the optode. By measuring the value of the excited state of the optical element it is possible to detect the neural activity of the brain of the patient under test.

It is also known in the art the use of the silicon photomultiplier as optical sensor.

In particular, a silicon photomultiplier generally consists of an array of single photon avalanche detectors (SPADs) with resistors or active quenching circuits in series. As known in the art, silicon photomultipliers are characterized by a very high gain, of the of the order of 107 electrons per photon, with, at the same time, low operation voltage, small size, high robustness and reliability, and low cost.

### Problem of the prior art

Unfortunately, the silicon photomultipliers have a very low linearity region. Namely, the value of the excited state of the silicon photomultiplier is linearly proportional to the incident optical power only for a limited range of values.

As known in the art, the highest signal-to-noise ratio occurs only for values of the excited state of the silicon photomultiplier comprised in the range of linearity and in particular close to the upper limit of the range. In other words, in order to exploit all the advantages of the silicon photomultiplier, it is strictly necessary that the value of the excited state of the silicon photomultiplier has to be comprised in the range of linearity and in particular close to the upper limit of the range. Consequently, it means that in order to use silicon photomultipliers it is necessary to select the region of operation of the silicon photomultiplier accurately.

Still unfortunately, the known fNIRS systems do not allow to selectively tune the range of operation of the used optical sensors. In fact, the response of the optical sensor in the fNIRS system depends on the position of the sensor on the head of the patient, on the physical distance between the optical sensor and the optical element of the same optode, on the property of the brain tissues, on the patient skin and hair color, etc. As a result, silicon photomultipliers can be used as optical sensors in fNIRS but with performances lower than their actual potential.

For example, "Wearable and modular functional near-infrared spectroscopy instrument with multidistance measurements at four wavelengths" by Wyser et alii describe a nfNIRS instrument. Said nfNIRS instrument comprises optode modules that comprise a four-wavelength light source, in particular LEDs, and a highly sensitive silicon photomultiplier detector. In detail, a voltage is supplied to the silicon photomultiplier. Said nfNIRS instrument comprises a control unit, connected to a personal computer, which performs all data processing steps and simultaneously acts as the interface between the computer and the optode modules. Acquired data, i.e. light emission and detection, are communicated to the control unit.

### Scope of the invention

In this context, the technical object of the present invention is to provide a system and a corresponding method for brain tissue analysis which overcome the drawbacks of the prior art.

Namely, the object of the present invention is to provide a system and a method for brain tissue analysis that allow using silicon photomultipliers with the highest signal-to-noise ratio.

The specified technical object and the specified aims are substantially achieved by a system and a corresponding method for brain tissue analysis comprising the technical characteristics described in one or more of the appended claims.

### Advantages of the invention

According to a preferred embodiment of the invention, it is possible to tune selectively the range of operation of the silicon photomultipliers so that the value of the excited state of the silicon photomultiplier is comprised in the range of linearity.

Namely, according to the preferred embodiment of the invention it is possible to tune the electrical power supplied to the optical element of the optode as a function of the position of the silicon photomultiplier on the head of a patient, the physical distance between the silicon photomultiplier and the optical element of the same optode and the properties of the tissue.

Still advantageously, the system and the method according to the present invention allows performing diffuse optical tomography (DOT) of the brain tissue of a patient. By exploiting all the advantages of the silicon photomultipliers, it is possible to increase the number of optodes positioned on the head of a patient, thus allowing to study the brain tissue at different depths.

### BRIEF DESCRIPTION OF THE DRAWINGS

Further characteristics and advantages of the present invention will appear more clearly from the indicative, and therefore non-limiting, description of a preferred but not exclusive embodiment of a system and a corresponding method for brain tissue analysis as illustrated in the enclosed drawings in which:

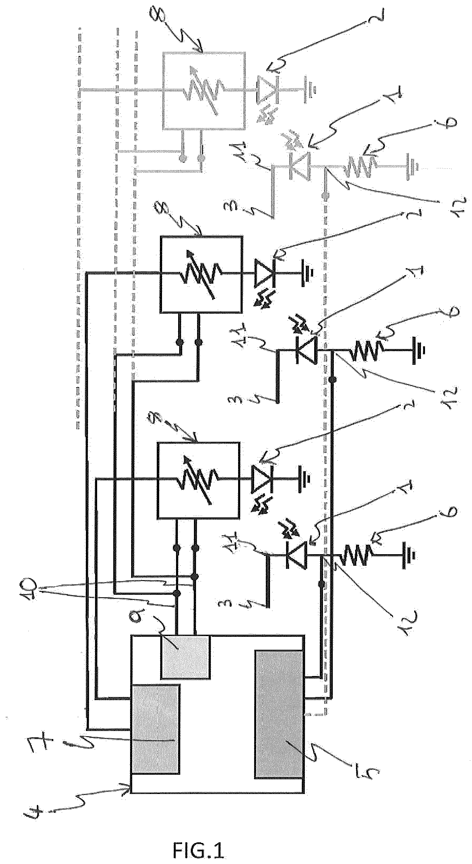

- Figure 1 is a schematic electric circuit representation of a portion of the system according to the present invention;

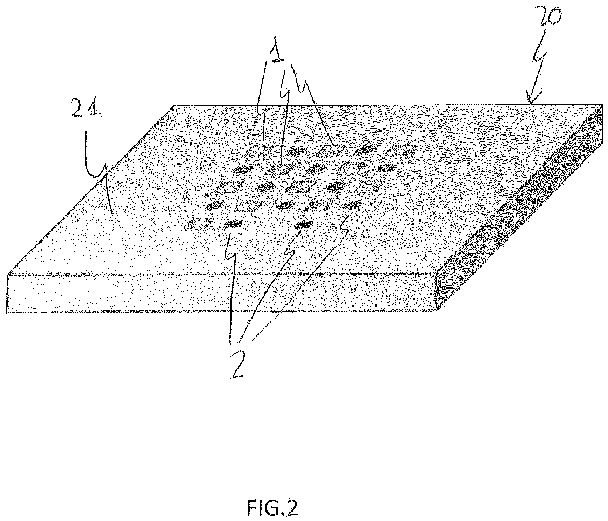

- Figure 2 is a perspective view of a plurality of silicon photomultipliers and optical elements disposed according to a predetermined pattern on a control body;

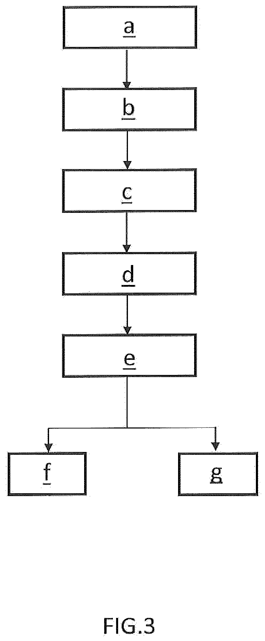

- Figure 3 is a flow chart describing the method according to the present invention.

### DETAILED DESCRIPTION

According to the present invention, a system for brain tissue analysis comprises at least one silicon photomultiplier 1, that is configured to change from a rest state to an excited state when excited by light.

The excited state of the silicon photomultiplier 1 corresponds to a linear state when the value of the excited state is comprised between a minimum linear value and a maximum linear value.

The value of the excited state depends on the value of the optical power illuminating the silicon photomultiplier 1. In other words, depending on the value of the optical power illuminating the silicon photomultiplier 1, the excited state of the silicon photomultiplier 1 corresponds or not to the linear state

The system comprises at least one optical element 2 comprising at least two emitting sources.

The number of the emitting sources can be at least two, but the system works also with a higher number of emitting sources such as three, four, five or more.

The emitting sources can be of the same type such as Led, Laser or fluorescence or can be a combination of different emitting sources, such as Led with a Laser or Led with fluorescence or Laser with fluorescence.

Each emitting source is configured to emit light at a given mean wavelength with full width half maximum at least less than 50% of the mean when the optical element 2 is supplied by an electrical power.

Preferably, the two emitting sources emit light at different mean wavelengths. More preferably, each emitting source emits light at a mean wavelength comprised in the range of 700-950 nm.

In the case, the emitting sources are LED's type then the given mean wavelength with full width half maximum is less than 25% of the mean, preferably less than 15%.

In the case, the emitting sources are fluorescence's type then the given mean wavelength with full width half maximum is less than 50% of the mean.

According to an aspect of the description, in the case the system 1 comprises two emitting sources (i.e. two LED), one of the two light mean wavelengths emitted must be lower and the other higher than a specific wavelength point, i.e. the so called Isosbestic point having a wavelength of about 800nm.

In the case the system 1 comprises more than two emitting sources then there could be one or more but not all emitting sources at mean wavelength lower than the Isosbestic point and the others higher than the Isosbestic point.

The system comprises a source of electric voltage 3 in signal communication with the silicon photomultiplier 1.

The source of electric voltage is configured to supply a given electric voltage to the silicon photomultiplier 1.

Once supplied by a value of electric voltage, the silicon photomultiplier 1 can change from the rest state to the excited state when excited by light.

The system comprises also a digital control and data elaboration unit 4 in signal communication with the silicon photomultiplier 1 and with the optical element 2.

The digital control and data elaboration unit 4 comprises a control unit configured to define an optical and electrical association between the optical element 2 and the silicon photomultiplier 1. In other words, the control unit is configured to define an optode.

The control unit is also configured to define a value of electrical power to be supplied to the optical element 2, while the digital control and data elaboration unit 4 is configured to supply the value of electrical power defined by the control unit to the optical element 2.

The system comprises a memory unit in signal communication with the digital control and data elaboration unit 4. The memory unit stores the minimum linear value and the maximum linear value of the linear state of the silicon photomultiplier 1 and it is configured to store values of the corresponding values of electrical power to be provided to the optical element 2. According to a preferred embodiment, the memory unit is integrated in the digital control and data elaboration unit 4.

The digital control and data elaboration unit 4 comprises a measuring unit 5 in signal communication with the silicon photomultiplier 1 and with the control unit. The measuring unit 5 is configured to measure a value of the excited state of the silicon photomultiplier 1 and to transmit said measured value of the excited state of the silicon photomultiplier 1 to the control unit.

According to an aspect, the control unit is configured to verify if said value of the excited state is comprised between the minimum linear value and the maximum linear value. In other words, the control unit is configured to compare the value of the excited state measured by the measuring unit 5 with the minimum and maximum linear value stored in the memory unit.

It is to point out that, in function of the result of the verification, the control unit is configured to perform two different operations.

First of all, the control unit is configured to change the value of the electrical power to be supplied to the optical element 2 when the value of the excited state of the silicon photomultiplier 1, measured by the measuring unit 5, is lower than the minimum linear value or greater than the maximum linear value.

Vice versa, the control unit is configured to store in the memory unit the value of the electrical power to be supplied to the optical element 2 when the value of the excited state of the silicon photomultiplier 1, measured by the measuring unit 5, is comprised between the minimum linear value and the maximum linear value.

Preferably, the control unit is configured to store in the memory unit the value of the electrical power to be supplied to the optical element 2 when the value of the excited state of the silicon photomultiplier 1 is comprised between the minimum and the maximum linear value but, at the same time, is very close to the maximum linear value. In fact, it is possible to detect the highest signal to noise ratio for values of the excited state of the silicon photomultiplier 1 closed to the maximum linear value.

According to a preferred embodiment of the system, the silicon photomultiplier 1 comprises a first 11 and a second terminal 12.

The source of electric voltage 3 is configured to supply the given electric voltage to the silicon photomultiplier 1 at the first terminal 11, while the second terminal 12 is connected to ground trough a control resistance 6. In other words, the silicon photomultiplier 1 is electrically connected in series to the control resistance 6.

The measuring unit 5 of the digital control and data elaboration unit 4 is configured to measure the value of the excited state of the silicon photomultiplier 1 by measuring the electric voltage difference between the second terminal 12 and the ground. In other words, the measuring unit 5 is configured to measure the value of the excited state of the silicon photomultiplier 1 by measuring the electric voltage difference across the control resistance.

In fact, depending on the value of the excited state of the silicon photomultiplier 1, the value of the electrical current flowing in the control resistance 6 will be different. Depending on the value of the electrical current flowing in the control resistance 6, the value of electrical voltage difference between the second terminal 12 and the ground will be different. In other words, a value of excited state of the silicon photomultiplier is translated into a corresponding value of electric voltage difference between the second terminal 12 and the ground.

According to a preferred embodiment of the system, the optical element 2 is connected to an electrical output 7 of the digital control and data elaboration unit 4 through a digital controlled dimmer 8.

The digital controlled dimmer 8 comprises a controllable impedance. In other words, the value of the impedance of the digital controlled dimmer 8 can be digitally controlled.

The digital controlled dimmer 8 is connected to an interface 9 of the digital control and data elaboration unit 4 through a BUS 10.

The digital control and data elaboration unit 4 is configured to supply the electrical power to the optical element 2 at the electrical output 7, while the control unit is configured to change the value of the electrical power to be supplied to the optical element 2 by regulating the digital controlled dimmer 8 through the BUS 10. In other words, the control unit is configured to change the value of electrical power to be supplied to the optical element 2 by regulating the value of the impedance of the digital controlled dimmer 8. In conclusion, the value of electrical power received by the optical element 2 depends on the value of electrical power supplied by the digital control and data elaboration unit 4 at the electrical output 7 but also on the value of the impedance of the digital controlled dimmer 8 controlled by the control unit.

According to a preferred embodiment, the system comprises a plurality of silicon photomultipliers 1 and a plurality of optical elements 2.

Each silicon photomultiplier 1 is in signal communication with the digital control and data elaboration unit 4 and with the source of electric voltage 3, while each optical element 2 is in signal communication with the digital control and data elaboration unit 4.

Preferably, all the silicon photomultipliers 1 are connected to ground through a respective control resistance 6 or by any equivalent device.

Still preferably, each optical element 2 is connected to its relative electrical output 7 of the digital control and data elaboration unit 4 through the respective digital controlled dimmer 8. Each digital controlled dimmer 8 is connected to the interface 9 of the digital control and data elaboration unit 4 through a respective BUS 10.

The source of electric voltage 3 is configured to supply the given electric voltage to each silicon photomultiplier 1. At the same time, the control unit is configured to sequentially choose one optical element 2 and to define an optical and electrical association between the chosen optical element 2 and at least one silicon photomultiplier 1 and to define a value of electrical power to be supplied to the chosen optical element 2.

As it will be better described hereinafter, the control unit is configured to sequentially associate one silicon photomultiplier 1 to one optical element 2 until all the silicon photomultipliers 1 have been associated to at least one optical element 2 and all the optical elements 2 have been associated to at least one silicon photomultiplier 1.

A method for brain tissue analysis, that uses the system already described, is another object of the present invention. This method comprises the step of disposing the silicon photomultipliers 1 and the optical elements 2 on the head of a patient according to a given geometrical pattern. Preferably, the silicon photomultipliers 1 and the optical elements 2 are disposed on the head of a patient in order to define a wide range of distances between the silicon photomultipliers 1 and the optical elements 2.

In fact, as it will be better described hereinafter, each distance between a silicon photomultiplier 1 and an optical element 2 corresponds approximately to a particular depth of analysis inside the brain tissue of the patient. Advantageously, exploiting a wide range of distances between the silicon photomultipliers 1 and the optical elements 2 allows to obtain a high amount of information from the brain tissue analysis.

Subsequently, the method comprises the biasing of all silicon photomultipliers by the source of electric voltage 3 with a given electric voltage, and the sequential repetition of the following steps until each silicon photomultiplier 1 has been associated to at least one optical element 2 and each optical element 2 has been associated to at least one silicon photomultiplier 1:
a) choosing, by the control unit, one optical element 2;
b) defining, by the control unit, an optical and electrical association of the chosen optical element 2 and at least one silicon photomultiplier 1;
c) defining, by the control unit, a value of electrical power to be supplied to the chosen optical element 2;
d) supplying, by the digital control and data elaboration unit 4, the value of electrical power defined by the control unit to the chosen optical element 2. Preferably, the electrical power is supplied by the digital control and data elaboration unit 4 at the electrical output 7. After supplying the electrical power to the optical element 2 of the association, the light emitted by the two emitting sources of the optical element 2 can diffuse through the brain tissue of the patient. Part of the light emitted by the optical element is absorbed by the brain tissue, while the remaining part of the light is scattered. Part of the backscattered light excites the silicon photomultipliers 1 of the association;
e) measuring, by the measuring unit 5, the value of the excited state of each silicon photomultiplier 1 of the association and verifying, by the control unit, if the value of the excited state of each silicon photomultiplier 1 of the association is comprised between the minimum and the maximum linear value of the linear state. Preferably, the measuring unit 5 measures the electric voltage difference between the second terminal 12 of each silicon photomultipliers 1 of the association and the ground;
f) changing, by the control unit, the value of electrical power to be supplied to the chosen optical element 2 if the value of the excited state of at least one silicon photomultiplier 1 of the association is not comprised between the minimum and maximum linear value of the linear state and repeating steps from d) to f). Preferably, the control unit changes the value of electrical power to be supplied to the optical element 2 by changing the value of the impedance of the digital control dimmer 8, while maintaining fixed the same value of electrical power supplied at the electrical output 7;
g) storing in the memory unit, by the control unit, the value of electrical power to be supplied to the chosen optical element 2 together with the corresponding association, if the value of the excited state of each silicon photomultiplier 1 of the association is comprised between the minimum and the maximum linear value of the linear state.

After the sequential repetition of steps from a) to g) the memory unit has stored all the optical and electrical associations defined by the control unit with the corresponding values of electrical power to be supplied to the optical element 2 of each association such that the excited state of all the silicon photomultipliers 1 of each association corresponds to a linear state.

According to one embodiment of the invention, the step of arranging the silicon photomultipliers 1 and the optical elements 2 on the head of a patient according to a given geometrical pattern comprises disposing the silicon photomultipliers 1 and the optical elements 2 on the head of a patient according to a checkerboard pattern.

According to an embodiment of the invention alternative to the previous one, the step of disposing the silicon photomultipliers 1 and the optical elements 2 on the head of a patient according to a given geometrical pattern comprises arranging the silicon photomultipliers 1 and the optical elements 2 on the head of a patient according to a honeycomb pattern.

Preferably, after disposing the silicon photomultipliers 1 and the optical elements 2 on the head of the patient according to a given geometrical pattern, the method comprises measuring and storing in the memory unit all the physical distances between each optical element 2 and all the silicon photomultipliers 1.

Preferably, the step of defining, by the control unit, an optical and electrical association of the chosen optical element 2 and at least one silicon photomultiplier 1 comprises defining all the physical distances between the chosen optical element 2 and each silicon photomultipliers 1. In other words, the method comprises selecting, by the control unit, all the values of physical distances associated to the chosen optical element 2 and stored in the memory unit.

In addition, the step of defining, by the control unit, an optical and electrical association of the chosen optical element 2 and at least one silicon photomultiplier 1 comprises choosing, by the control unit, a value of physical distances among all the defined values.

Subsequently, the step of defining, by the control unit, an optical and electrical association of the chosen optical element 2 and at least one silicon photomultiplier 1 comprises defining, by the control unit, an association between the chosen optical element 2 and all the silicon photomultipliers 1 spaced apart from the chosen optical element 2 by the value of physical distance chosen in the previous step. In fact, the distance between an optical element 2 and a silicon photomultiplier 1 approximately corresponds to a particular depth of analysis in the brain tissue. For example, a distance D between an optical element 2 and a silicon photomultiplier 1 corresponds to a depth of analysis inside the brain tissue nearly equal to D/2. In other words, given a distance D between an optical element 2 and a silicon photomultiplier 1, the information that can be obtained from the excited state of said silicon photomultiplier 1 regards the brain tissue approximately located at a depth D/2 and in the middle point between the silicon photomultiplier 1 and the optical element 2. In other words, the method comprises associating, by the control unit, to the chosen optical element 2 all the silicon photomultipliers 1 that bring information regarding the same depth of the brain tissue of the patient.

Finally, the step of defining, by the control unit, an optical and electrical association of the chosen optical element 2 and at least one silicon photomultiplier 1 comprises storing, by the control unit, in the memory unit the association defined at the previous step together with the corresponding value of physical distance between the optical element 2 and each silicon photomultiplier 1 of the association.

Preferably, after the sequential repetition of steps from a) to g), the method comprises defining, by the control unit, at least one group of associations, each association of the same group is characterized by the same physical distance between the optical element 2 and each silicon photomultipliers 1 of the association. In other words, the method comprises grouping all the associations that bring information regarding the same depth of the brain tissue. In still other words, the method comprises grouping all the associations that bring information regarding the portion of the brain tissue that is located at the same depth from the external surface of the head.

Subsequently, the method comprises storing in the memory unit, by the control unit, each group of associations.

After defining at least one group of association, the method comprises the sequential activation of each group of associations by supplying, by the digital control and data elaboration unit 4, each optical element 2 of each association of the same group with the corresponding value of electrical power defined by the control unit. In other words, each optical element 2 of each association of the same group is supplied with the corresponding value of electrical power defined after sequential repetition of steps from a) to g). After the activation of each group of association, the light diffuses in the brain tissue of the patient and part of the backscattered light excites all the silicon photomultipliers 1 of the associations of the same group.

Subsequently, the method comprises measuring, by the measuring unit 5, and storing in the memory unit, by the control unit, after the activation of each group of associations, an operative value of the excited state of each silicon photomultiplier 1 of each association of the same group.

Then, the method comprises removing the optical elements 2 and the silicon photomultipliers 1 from the head of the patient.

After removing the optical elements 2 and the silicon photomultipliers 1 from the head of the patient, the method comprises providing a control body 20. Preferably, the control body 20 is a parallelepiped made of one or more materials with optical constants similar to the scalp, skull and brain tissues.

The method comprises also disposing the optical elements 2 and the silicon photomultipliers 1 on an upper surface 21 of the control body 20 according to the same geometrical pattern used for disposing the optical elements 2 and the silicon photomultipliers 1 on the head of a patient.

After disposing the optical elements 2 and the silicon photomultiplier 1 on the upper surface of the control body 20, and after supplying by the source of electric voltage 3 each silicon photomultiplier 1 with a given voltage, the method comprises the sequential activation of each group of associations by supplying by the digital control and data elaboration unit 4 each optical element 2 of each association of the same group with the same value of electrical power supplied to the optical elements 2 when positioned on the head of a patient. After activating each group of associations, light emitted by the optical elements 2 can diffuse through the control body 20 and part of backscattered light excites the silicon photomultiplier 1 of each association of the same group.

Then, the method comprises measuring, by the measuring unit 5, and storing in the memory unit, by the control unit, after the activation of each group of associations, a rest value of the excited state of each silicon photomultiplier 1 of each association of the same group.

After having activated all groups of association on the control body 20, the memory unit stores the operative value and the rest value of the excited state for each silicon photomultiplier 1 of each association of each group of association. More in details, for each silicon photomultiplier 1 the memory unit has stored a number of couples of operative and test values equal to the number of associations to which the silicon photomultiplier 1 belongs.

Lastly, the method comprises elaborating, by the control unit, the operative value and the rest value of the excited state of each silicon photomultiplier 1 of each association of each group of associations for defining a corresponding updated value of the excited state. Advantageously, defining an updated value of the excited state allows eliminating measurement errors that can arise when the silicon photomultipliers 1 are positioned on the head of the patient.

According to one embodiment of the invention, the step of elaborating, by the control unit, the operative value and the rest value of the excited state of each silicon photomultiplier 1 of each association of each group of association for defining a corresponding updated value of the excited state comprises defining, by the control unit, the updated value of the excited state of each silicon photomultiplier 1 of each association of each group of association as the division between the corresponding operative value of the excited state and the corresponding rest value of the excited state.

According to another embodiment of the invention alternative to the previous one, the step of elaborating, by the control unit, the operative value and the rest value of the excited state of each silicon photomultiplier 1 of each association of each group of association for defining a corresponding updated value of the excited state comprises defining, by the control unit, an updated value of the excited state of each silicon photomultiplier 1 of each association of each group of association as the subtraction between the corresponding operative value of the excited state and the corresponding rest value of the excited state.

## Claims

1. System for brain tissue analysis, said system comprising:
- at least one silicon photomultiplier (1) being configured to change from a rest state to an excited state when excited by light, said excited state corresponding to a linear state when the value of the excited state is comprised between a minimum linear value and a maximum linear value;
- at least one optical element (2) comprising at least two emitting sources, each emitting source being configured to emit light at a given mean wavelength with full width half maximum at least less than 50% of the mean when the optical element is supplied by an electrical power;
- a source of electric voltage (3) in signal communication with the silicon photomultiplier (1), said source of electric voltage being configured to supply a given electric voltage to the silicon photomultiplier (1);
- a digital control and data elaboration unit (4) in signal communication with the silicon photomultiplier (1) and with the optical element (2), said digital control and data elaboration unit (4) comprising a control unit configured to define an optical and electrical association between the optical element (2) and the silicon photomultiplier (1) and to define a value of electrical power to be supplied to the optical element (2), the digital control and data elaboration unit (4) being configured to supply the value of electrical power defined by the control unit to the optical element (2);
- a memory unit in signal communication with the digital control and data elaboration unit (4), said memory unit having stored the minimum linear value and the maximum linear value of the linear state of the silicon photomultiplier (1) and being configured to store values of electrical power to be supplied to the optical elements (2);
- in which the digital control and data elaboration unit (4) comprises a measuring unit (5) in signal communication with the silicon photomultiplier (1) and with the control unit, said measuring unit (5) being configured to measure a value of the excited state of the silicon photomultiplier (1);
**characterized in that:**
- the control unit is configured to verify if said value of the excited state is comprised between the minimum linear value and the maximum linear value;
- the control unit is configured to change the value of electrical power to be supplied to the optical element (2) when the value of the excited state of the silicon photomultiplier (1), measured by the measuring unit (5), is lower than the minimum linear value or larger than the maximum linear value;
- the control unit is configured to store in the memory unit the value of the electrical power to be supplied to the optical element (2) when the value of the excited state of the silicon photomultiplier (1), measured by the measuring unit (5), is comprised between the minimum linear value and the maximum linear value.

2. System according to claim 1, in which the silicon photomultiplier (1) comprises a first (11) and a second terminal (12), the source of electric voltage (3) being configured to supply the given electric voltage to the silicon photomultiplier (1) at the first terminal (11), said second terminal (12) being connected to ground trough a control resistance (6), the measuring unit (5) of the digital control and data elaboration unit (4) being configured to measure the value of the excited state of the silicon photomultiplier (1) by measuring the electric voltage difference between the second terminal (12) and the ground.

3. System according to claim 1 or 2, in which the optical element (2) is connected to an electrical output (7) of the digital control and data elaboration unit (4) through a digital controlled dimmer (8), said digital controlled dimmer (8) being connected to an interface (9) of the digital control and data elaboration unit (4) through a BUS (10), the digital control and data elaboration unit (4) being configured to supply the electrical power to the optical element (2) at the electrical output (7), the control unit being configured to change the value of the electrical power to be supplied to the optical element (2) by regulating the digital controlled dimmer (8) through the BUS (10).

4. System according to any claim 1 to 3, in which:
- said circuit comprises a plurality of silicon photomultipliers (1) and a plurality of optical elements (2), each silicon photomultiplier (1) being in signal communication with the digital control and data elaboration unit (4) and with the source of electric voltage (3), each optical element (2) being in signal communication with the digital control and data elaboration unit (4);
- the source of electric voltage (3) being configured to supply the given electric voltage to each silicon photomultiplier (1);
- the control unit is configured to sequentially choose one optical element (2) and to define an optical and electrical association between the chosen optical element (2) and at least one silicon photomultiplier (1) and to define a value of electrical power to be supplied to the chosen optical element (2);
- the digital control and data elaboration unit (4) being configured to supply the value of electrical power defined by the control unit to the chosen optical element (2) and the measuring unit (5) being configured to measure the value of the excited state of the silicon photomultipliers (1) of the association;
- said control unit being configured to change the value of the electrical power to be supplied to the chosen optical element (2) when the value of the excited state of at least one silicon photomultiplier (1) of the association is lower than the minimum linear value or larger than the maximum linear value of the linear state;
- said control unit being configured to store in the memory unit the value of electrical power to be supplied to the chosen optical element (2) when the value of the excited state of each silicon photomultiplier (1) of the association is comprised between the minimum and the maximum linear value of the linear state.

5. System according to any claim 1 to 4, in which the at least two emitting sources can be of the same of different type.

6. System according to any claim 1 to 5, in which one of the two light mean wavelengths emitted must be lower and the other higher than a specific wavelength point.

7. Method for brain tissue analysis comprising the following steps:
- providing a system according to any claim 1-6;
- disposing the silicon photomultipliers (1) and the optical elements (2) on the head of a patient according to a given geometrical pattern;
- sequential repetition of the following steps until each silicon photomultiplier (1) has been associated to at least one optical element (2) and each optical element (2) has been associated to at least one silicon photomultiplier (1):
a) choosing, by the control unit, one optical element (2);
b) defining, by the control unit, an optical and electrical association of the chosen optical element (2) and at least one silicon photomultiplier (1);
c) defining, by the control unit, a value of electrical power to be supplied to the chosen optical element (2);
d) supplying, by the digital control and data elaboration unit (4), the value of electrical power defined by the control unit to the chosen optical element (2);
e) measuring, by the measuring unit (5), the value of the excited state of each silicon photomultiplier (1) of the association and verifying, by the control unit, if the value of the excited state of each silicon photomultiplier (1) of the association is comprised between the minimum and the maximum linear value of the linear state;
f) changing, by the control unit, the value of electrical power to be supplied to the chosen optical element (2) if the value of the excited state of at least one silicon photomultiplier (1) of the association is not comprised between the minimum and the maximum linear value of the linear state and repeating steps from d) to f);
g) storing in the memory unit, by the control unit, the value of electrical power to be supplied to the chosen optical element (2) together with the corresponding association, if the value of the excited state of each silicon photomultiplier (1) of the association is comprised between the minimum and the maximum linear value of the linear state.

8. Method according to claim 7, in which the step of disposing the silicon photomultipliers (1) and the optical elements (2) on the head of a patient according to a given geometrical pattern comprises:
- disposing the silicon photomultipliers (1) and the optical elements (2) on the head of a patient according to a checkerboard pattern; or
- disposing the silicon photomultipliers (1) and the optical elements (2) on the head of a patient according to a honeycomb pattern.

9. Method according to claim 7 or 8, in which the step of defining, by the control unit, an optical and electrical association of the chosen optical element (2) and at least one silicon photomultiplier (1) comprises:
- defining all the physical distances between the chosen optical element (2) and each silicon photomultipliers (1);
- choosing, by the control unit, a value of physical distances among all the defined values;
- defining, by the control unit, an association between the chosen optical element (2) and all the silicon photomultipliers (1) spaced apart from the chosen optical element (2) by the value of physical distance chosen in the previous step;
- storing, by the control unit, in the memory unit the association defined at the previous step together with the corresponding value of physical distance between the optical element (2) and each silicon photomultiplier (1) of the association.

10. Method according to claim 9, in which, after the sequential repetition of steps from a) to g), the method comprises:
- defining, by the control unit, at least one group of associations, each association of the same group being **characterized by** the same physical distance between the optical element (2) and each silicon photomultiplier (1) of the association;
- storing in the memory unit, by the control unit, each group of associations;

11. Method according to claim 10, in which the method comprises the additional steps of:
- sequential activation of each group of associations by supplying, by the digital control and data elaboration unit (4), each optical element (2) of each association of the same group with the corresponding value of electrical power defined by the control unit;
- measuring, by the measuring unit (5), and storing in the memory unit, by the control unit, after the activation of each group of associations, an operative value of the excited state of each silicon photomultiplier (1) of each association of the same group;
- removing all the optical elements (2) and the silicon photomultipliers (1) from the head of the patient;
- providing a control body (20);
- disposing the optical elements (2) and the silicon photomultipliers (1) on an upper surface (21) of the control body (20) according to the same geometrical pattern used for disposing the optical elements (2) and the silicon photomultipliers (1) on the head of a patient;
- sequential activation of each group of associations by supplying, by the digital control and data elaboration unit (4), each optical element (2) of each association of the same group with the same value of electrical power supplied to the optical elements (2) when positioned on the head of a patient;
- measuring, by the measuring unit (5), and storing in the memory unit, by the control unit, after the activation of each group of associations, a rest value of the excited state of each silicon photomultiplier (1) of each association of the same group;
- elaborating, by the control unit, the operative value and the rest value of the excited state of each silicon photomultiplier (1) of each association of each group of association for defining a corresponding updated value of the excited state.

12. Method according to claim 11, in which the step of elaborating, by the control unit, the operative value and the rest value of the excited state of each silicon photomultiplier (1) of each association of each group of association for defining a corresponding updated value of the excited state comprises:
- defining, by the control unit, the updated value of the excited state of each silicon photomultiplier (1) of each association of each group of association as the division between the corresponding operative value of the excited state and the corresponding rest value of the excited state; or
- defining, by the control unit, an updated value of the excited state of each silicon photomultiplier (1) of each association of each group of association as the subtraction between the corresponding operative value of the excited state and the corresponding rest value of the excited state.

## Patentansprüche

1. System zur Analyse von Hirngewebe, wobei das System Folgendes umfasst:
- mindestens einen Silicium-Photovervielfacher (1), der so konfiguriert ist, dass er von einem Ruhezustand in einen angeregten Zustand übergeht, wenn er durch Licht angeregt wird, wobei der angeregte Zustand einem linearen Zustand entspricht, wenn der Wert des angeregten Zustands zwischen einem minimalen linearen Wert und einem maximalen linearen Wert liegt;
- mindestens ein optisches Element (2), das mindestens zwei Emissionsquellen umfasst, wobei jede Emissionsquelle so konfiguriert ist, dass sie Licht bei einer gegebenen mittleren Wellenlänge mit einer Halbwertsbreite von mindestens 50 % des Mittelwerts emittiert, wenn dem optischen Element eine elektrische Leistung zugeführt wird;
- eine elektrische Spannungsquelle (3) in Signalkommunikation mit dem Silicium-Photovervielfacher (1), wobei die elektrische Spannungsquelle so konfiguriert ist, dass sie dem Silicium-Photovervielfacher (1) eine bestimmte elektrische Spannung zuführt;
- eine digitale Steuer- und Datenverarbeitungseinheit (4) in Signalkommunikation mit dem Silicium-Photovervielfacher (1) und mit dem optischen Element (2), wobei die digitale Steuer- und Datenverarbeitungseinheit (4) eine Steuereinheit umfasst, die so konfiguriert ist, dass sie eine optische und elektrische Zuordnung zwischen dem optischen Element (2) und dem Silicium-Photovervielfacher (1) definiert und einen Wert der dem optischen Element (2) zuzuführenden elektrischen Leistung definiert, wobei die digitale Steuer- und Datenverarbeitungseinheit (4) so konfiguriert ist, dass sie den von der Steuereinheit definierten Wert der elektrischen Leistung dem optischen Element (2) zuführt;
- eine Speichereinheit in Signalkommunikation mit der digitalen Steuer- und Datenverarbeitungseinheit (4), wobei die Speichereinheit den minimalen linearen Wert und den maximalen linearen Wert des linearen Zustands des Silicium-Photovervielfachers (1) gespeichert hat und so konfiguriert ist, dass sie Werte der den optischen Elementen (2) zuzuführenden elektrischen Leistung speichert;
- wobei die digitale Steuer- und Datenverarbeitungseinheit (4) eine Messeinheit (5) in Signalkommunikation mit dem Silicium-Photovervielfacher (1) und mit der Steuereinheit umfasst, wobei die Messeinheit (5) so konfiguriert ist, dass sie einen Wert des angeregten Zustands des Silicium-Photovervielfachers (1) misst; **dadurch gekennzeichnet, dass:**
- die Steuereinheit so konfiguriert ist, dass sie prüft, ob der Wert des angeregten Zustands zwischen dem minimalen linearen Wert und dem maximalen linearen Wert liegt;
- die Steuereinheit so konfiguriert ist, dass sie den Wert der dem optischen Element (2) zuzuführenden elektrischen Leistung ändert, wenn der von der Messeinheit (5) gemessene Wert des angeregten Zustands des Silicium-Photovervielfachers (1) kleiner als der minimale lineare Wert oder größer als der maximale lineare Wert ist;
- die Steuereinheit so konfiguriert ist, dass sie in der Speichereinheit den Wert der dem optischen Element (2) zuzuführenden elektrischen Leistung speichert, wenn der von der Messeinheit (5) gemessene Wert des angeregten Zustands des Silicium-Photovervielfachers (1) zwischen dem minimalen linearen Wert und dem maximalen linearen Wert liegt.

2. System nach Anspruch 1, wobei der Silicium-Photovervielfacher (1) einen ersten (11) und einen zweiten Anschluss (12) umfasst, wobei die elektrische Spannungsquelle (3) so konfiguriert ist, dass sie die gegebene elektrische Spannung dem Silicium-Photovervielfacher (1) am ersten Anschluss (11) zuführt, wobei der zweite Anschluss (12) über einen Steuerwiderstand (6) mit Masse verbunden ist, wobei die Messeinheit (5) der digitalen Steuer- und Datenverarbeitungseinheit (4) so konfiguriert ist, dass sie den Wert des angeregten Zustands des Silicium-Photovervielfachers (1) durch Messen der elektrischen Spannungsdifferenz zwischen dem zweiten Anschluss (12) und der Masse misst.

3. System nach Anspruch 1 oder 2, wobei das optische Element (2) über einen digital gesteuerten Dimmer (8) mit einem elektrischen Ausgang (7) der digitalen Steuer- und Datenverarbeitungseinheit (4) verbunden ist, wobei der digital gesteuerte Dimmer (8) über einen BUS (10) mit einer Schnittstelle (9) der digitalen Steuer- und Datenverarbeitungseinheit (4) verbunden ist, wobei die digitale Steuer- und Datenverarbeitungseinheit (4) so konfiguriert ist, dass sie dem optischen Element (2) am elektrischen Ausgang (7) elektrische Leistung zuführt, wobei die Steuereinheit so konfiguriert ist, dass sie den Wert der dem optischen Element (2) zuzuführenden elektrischen Leistung durch Regulierung des digital gesteuerten Dimmers (8) über den BUS (10) ändert.

4. System nach einem der Ansprüche 1 bis 3, wobei:
- die Schaltung eine Vielzahl von Silicium-Photovervielfachern (1) und eine Vielzahl von optischen Elementen (2) umfasst, wobei jeder Silicium-Photovervielfacher (1) in Signalkommunikation mit der digitalen Steuer- und Datenverarbeitungseinheit (4) und mit der elektrischen Spannungsquelle (3) steht, wobei jedes optische Element (2) in Signalkommunikation mit der digitalen Steuer- und Datenverarbeitungseinheit (4) steht;
- wobei die elektrische Spannungsquelle (3) so konfiguriert ist, dass sie die gegebene elektrische Spannung jedem Silicium-Photovervielfacher (l) zuführt;
- die Steuereinheit so konfiguriert ist, dass sie sequentiell ein optisches Element (2) wählt und eine optische und elektrische Zuordnung zwischen dem gewählten optischen Element (2) und mindestens einem Silicium-Photovervielfacher (1) definiert und einen Wert der dem gewählten optischen Element (2) zuzuführenden elektrischen Leistung definiert,
- wobei die digitale Steuer- und Datenverarbeitungseinheit (4) so konfiguriert ist, dass sie den von der Steuereinheit definierten Wert der elektrischen Leistung dem gewählten optischen Element (2) zuführt, und die Messeinheit (5) so konfiguriert ist, dass sie den Wert des angeregten Zustands der Silicium-Photovervielfacher (1) der Zuordnung misst;
- wobei die Steuereinheit so konfiguriert ist, dass sie den Wert der dem gewählten optischen Element (2) zuzuführenden elektrischen Leistung ändert, wenn der Wert des angeregten Zustands von mindestens einem Silicium-Photovervielfacher (1) der Zuordnung kleiner als der minimale lineare Wert oder größer als der maximale lineare Wert des linearen Zustands ist;
- wobei die Steuereinheit so konfiguriert ist, dass sie in der Speichereinheit den Wert der dem gewählten optischen Element (2) zuzuführenden elektrischen Leistung speichert, wenn der Wert des angeregten Zustands jedes Silicium-Photovervielfachers (1) der Zuordnung zwischen dem minimalen und dem maximalen linearen Wert des linearen Zustands liegt.

5. System nach einem der Ansprüche 1 bis 4, wobei die mindestens zwei emittierenden Quellen gleichen oder unterschiedlichen Typs sein können.

6. System nach einem der Ansprüche 1 bis 5, wobei eine der beiden mittleren Wellenlängen des emittierten Lichts niedriger und die andere höher als ein bestimmter Wellenlängenpunkt sein muss.

7. Verfahren zur Analyse von Hirngewebe, umfassend die folgenden Schritte:
- Bereitstellen eines Systems nach einem der Ansprüche 1-6;
- Anordnen der Silicium-Photovervielfacher (1) und der optischen Elemente (2) auf dem Kopf eines Patienten nach einem bestimmten geometrischen Muster;
- sequentielles Wiederholen der folgenden Schritte, bis jeder Silicium-Photovervielfacher (1) mindestens einem optischen Element (2) zugeordnet ist und jedes optische Element (2) mindestens einem Silicium-Photovervielfacher (1) zugeordnet ist:
a) Wählen eines optischen Elements (2) durch die Steuereinheit;
b) Definieren, durch die Steuereinheit, einer optischen und elektrischen Zuordnung zwischen dem gewählten optischen Element (2) und mindestens einem Silicium-Photovervielfacher (l);
c) Definieren, durch die Steuereinheit, eines Wertes der dem gewählten optischen Element (2) zuzuführenden elektrischen Leistung;
d) Zuführen, durch die digitale Steuer- und Datenverarbeitungseinheit (4), des von der Steuereinheit definierten Wertes der elektrischen Leistung an das ausgewählte optische Element (2);
e) Messen, durch die Messeinheit (5), des Wertes des angeregten Zustands jedes Silicium-Photovervielfachers (1) der Zuordnung und Überprüfen, durch die Steuereinheit, ob der Wert des angeregten Zustands jedes Silicium-Photovervielfachers (1) der Zuordnung zwischen dem minimalen und dem maximalen linearen Wert des linearen Zustands liegt;
f) Ändern, durch die Steuereinheit, des Wertes der dem gewählten optischen Element (2) zuzuführenden elektrischen Leistung, wenn der Wert des angeregten Zustands von mindestens einem Silicium-Photovervielfacher (1) der Zuordnung nicht zwischen dem minimalen und dem maximalen linearen Wert des linearen Zustands liegt, und Wiederholen der Schritte von d) bis f);
g) Speichern, in der Speichereinheit, durch die Steuereinheit, des Wertes der dem gewählten optischen Element (2) zuzuführenden elektrischen Leistung zusammen mit der entsprechenden Zuordnung, wenn der Wert des angeregten Zustands jedes Silicium-Photovervielfachers (1) der Zuordnung zwischen dem minimalen und dem maximalen linearen Wert des linearen Zustands liegt.

8. Verfahren nach Anspruch 7, wobei der Schritt des Anordnens der Silicium-Photovervielfacher (1) und der optischen Elemente (2) auf dem Kopf eines Patienten gemäß einem vorgegebenen geometrischen Muster umfasst:
- Anordnen der Silicium-Photovervielfacher (1) und der optischen Elemente (2) auf dem Kopf eines Patienten nach einem Schachbrettmuster; oder
- Anordnen der Silicium-Photovervielfacher (1) und der optischen Elemente (2) auf dem Kopf eines Patienten nach einem Wabenmuster.

9. Verfahren nach Anspruch 7 oder 8, wobei der Schritt des Definierens, durch die Steuereinheit, einer optischen und elektrischen Zuordnung des gewählten optischen Elements (2) und mindestens eines Silicium-Photovervielfachers (1) umfasst:
- Definieren aller räumlichen Distanzen zwischen dem gewählten optischen Element (2) und jedem Silicium-Photovervielfacher (1);
- Wählen, durch die Steuereinheit, eines Wertes der räumlichen Distanzen unter allen definierten Werten;
- Definieren, durch die Steuereinheit, einer Zuordnung zwischen dem gewählten optischen Element (2) und allen Silicium-Photovervielfachern (1), die von dem gewählten optischen Element (2) um den Wert der im vorherigen Schritt gewählten räumlichen Distanz beabstandet sind;
- Speichern, durch die Steuereinheit in der Speichereinheit, der im vorherigen Schritt definierten Zuordnung zusammen mit dem entsprechenden Wert der räumlichen Distanz zwischen dem optischen Element (2) und jedem Silicium-Photovervielfacher (1) der Zuordnung.

10. Verfahren nach Anspruch 9, wobei das Verfahren nach der sequentiellen Wiederholung der Schritte a) bis g) umfasst:
- Definieren, durch die Steuereinheit, von mindestens einer Gruppe von Zuordnungen, wobei jede Zuordnung derselben Gruppe durch dieselbe räumliche Distanz zwischen dem optischen Element (2) und jedem Silicium-Photovervielfacher (1) der Zuordnung gekennzeichnet ist;
- Speichern, in der Speichereinheit, durch die Steuereinheit, jeder Gruppe von Zuordnungen;

11. Verfahren nach Anspruch 10, wobei das Verfahren die folgenden zusätzlichen Schritte umfasst:
- sequentielles Aktivieren jeder Gruppe von Zuordnungen durch Zuführen, durch die digitale Steuer- und Datenverarbeitungseinheit (4), des entsprechenden, von der Steuereinheit definierten Wertes elektrischer Leistung an jedes optische Element (2) jeder Zuordnung derselben Gruppe;
- Messen, durch die Messeinheit (5), und Speichern in der Speichereinheit, durch die Steuereinheit, nach Aktivierung jeder Gruppe von Zuordnungen, eines operativen Werts des angeregten Zustands jedes Silicium-Photovervielfachers (1) jeder Zuordnung derselben Gruppe;
- Entfernen aller optischen Elemente (2) und Silicium-Photovervielfacher (1) vom Kopf des Patienten;
- Bereitstellen eines Steuerkörpers (20);
- Anordnen der optischen Elemente (2) und der Silicium-Photovervielfacher (1) auf einer oberen Fläche (21) des Steuerkörpers (20) nach demselben geometrischen Muster, das für das Anordnen der optischen Elemente (2) und der Silicium-Photovervielfacher (1) auf dem Kopf eines Patienten verwendet wurde;
- sequentielles Aktivieren jeder Gruppe von Zuordnungen durch Zuführen, durch die digitale Steuer- und Datenverarbeitungseinheit (4), an jedes optische Element (2) jeder Zuordnung derselben Gruppe, desselben Wertes elektrischer Leistung, die den optischen Elementen (2) zugeführt wurde, als sie auf dem Kopf eines Patienten positioniert waren;
- Messen, durch die Messeinheit (5), und Speichern in der Speichereinheit, durch die Steuereinheit, nach Aktivierung jeder Gruppe von Zuordnungen, eines Ruhewerts des angeregten Zustands jedes Silicium-Photovervielfachers (1) jeder Zuordnung derselben Gruppe;
- Ausarbeiten, durch die Steuereinheit, des operativen Wertes und des Ruhewerts des angeregten Zustands jedes Silicium-Photovervielfachers (1) jeder Zuordnung jeder Gruppe von Zuordnungen, um einen entsprechenden aktualisierten Wert des angeregten Zustands zu definieren.

12. Verfahren nach Anspruch 11, wobei der Schritt des Ausarbeitens, durch die Steuereinheit, des operativen Wertes und des Ruhewerts des angeregten Zustands jedes Silicium-Photovervielfachers (1) jeder Zuordnung jeder Gruppe von Zuordnungen zum Definieren eines entsprechenden aktualisierten Wertes des angeregten Zustands umfasst:
- Definieren, durch die Steuereinheit, des aktualisierten Wertes des angeregten Zustands jedes Silicium-Photovervielfachers (1) jeder Zuordnung jeder Gruppe von Zuordnungen als Division zwischen dem entsprechenden operativen Wert des angeregten Zustands und dem entsprechenden Ruhewert des angeregten Zustands; oder
- Definieren, durch die Steuereinheit, eines aktualisierten Wertes des angeregten Zustands jedes Silicium-Photovervielfachers (1) jeder Zuordnung jeder Gruppe von Zuordnungen als Subtraktion zwischen dem entsprechenden operativen Wert des angeregten Zustands und dem entsprechenden Ruhewert des angeregten Zustands.

## Revendications

1. Système d'analyse de tissu cérébral, ledit système comprenant :
- au moins un photomultiplicateur en silicium (1) qui est configuré pour passer d'un état de repos à un état excité lorsqu'il est excité par de la lumière, ledit état excité correspondant à un état linéaire lorsque la valeur de l'état excité est comprise entre une valeur linéaire minimale et une valeur linéaire maximale ;
- au moins un élément optique (2) comprenant au moins deux sources d'émission, chaque source d'émission étant configurée pour émettre une lumière à une longueur d'onde moyenne donnée ayant une largeur totale à mi-hauteur au moins inférieure à 50 % de la moyenne lorsque l'élément optique est alimenté par une énergie électrique ;
- une source de tension électrique (3) en communication de signal avec le photomultiplicateur en silicium (1), ladite source de tension électrique étant configurée pour alimenter, avec une tension électrique donnée, le photomultiplicateur en silicium (1) ;
- une unité de commande numérique et d'élaboration de données (4) en communication de signal avec le photomultiplicateur en silicium (1) et avec l'élément optique (2), ladite unité de commande numérique et d'élaboration de données (4) comprenant une unité de commande configurée pour définir une association optique et électrique entre l'élément optique (2) et le photomultiplicateur en silicium (1) et pour définir une valeur d'énergie électrique devant alimenter l'élément optique (2), l'unité de commande numérique et d'élaboration de données (4) étant configurée pour alimenter, avec la valeur d'énergie électrique définie par l'unité de commande, l'élément optique (2) ;
- une unité de mémoire en communication de signal avec l'unité de commande numérique et d'élaboration de données (4), ladite unité de mémoire ayant stocké la valeur linéaire minimale et la valeur linéaire maximale de l'état linéaire du photomultiplicateur en silicium (1) et étant configurée pour stocker des valeurs d'énergie électrique devant alimenter les éléments optiques (2) ;
- dans lequel l'unité de commande numérique et d'élaboration de données (4) comprend une unité de mesure (5) en communication de signal avec le photomultiplicateur en silicium (1) et avec l'unité de commande, ladite unité de mesure (5) étant configurée pour mesurer une valeur de l'état excité du photomultiplicateur en silicium (1) ;
**caractérisé en ce que :**
- l'unité de commande est configurée pour vérifier si ladite valeur de l'état excité est comprise entre la valeur linéaire minimale et la valeur linéaire maximale ;
- l'unité de commande est configurée pour modifier la valeur d'énergie électrique devant alimenter l'élément optique (2) lorsque la valeur de l'état excité du photomultiplicateur en silicium (1), mesurée par l'unité de mesure (5), est inférieure à la valeur linéaire minimale ou supérieure à la valeur linéaire maximale ;
- l'unité de commande est configurée pour stocker, dans l'unité de mémoire, la valeur de l'énergie électrique devant alimenter l'élément optique (2) lorsque la valeur de l'état excité du photomultiplicateur en silicium (1), mesurée par l'unité de mesure (5), est comprise entre la valeur linéaire minimale et la valeur linéaire maximale.

2. Système selon la revendication 1, dans lequel le photomultiplicateur en silicium (1) comprend une première (11) et une seconde borne (12), la source de tension électrique (3) étant configurée pour alimenter, avec la tension électrique donnée, le photomultiplicateur en silicium (1) au niveau de la première borne (11), ladite second borne (12) étant connectée à la masse par le biais d'une résistance de commande (6), l'unité de mesure (5) de l'unité de commande numérique et d'élaboration de données (4) étant configurée pour mesurer la valeur de l'état excité du photomultiplicateur en silicium (1) par la mesure de la différence de tension électrique entre la seconde borne (12) et la masse.

3. Système selon la revendication 1 ou 2, dans lequel l'élément optique (2) est connecté à une sortie électrique (7) de l'unité de commande numérique et d'élaboration de données (4) par le biais d'un variateur à commande numérique (8), ledit variateur à commande numérique (8) étant connecté à une interface (9) de l'unité de commande numérique et d'élaboration de données (4) par le biais d'un BUS (10), l'unité de commande numérique et d'élaboration de données (4) étant configurée pour alimenter, avec l'énergie électrique, l'élément optique (2) au niveau de la sortie électrique (7), l'unité de commande étant configurée pour modifier la valeur de l'énergie électrique devant alimenter l'élément optique (2) par la régulation du variateur à commande numérique (8) par le biais du BUS (10).

4. Système selon une quelconque revendication 1 à 3, dans lequel :
- ledit circuit comprend une pluralité de photomultiplicateurs en silicium (1) et une pluralité d'éléments optiques (2), chaque photomultiplicateur en silicium (1) étant en communication de signal avec l'unité de commande numérique et d'élaboration de données (4) et avec la source de tension électrique (3), chaque élément optique (2) étant en communication de signal avec l'unité de commande numérique et d'élaboration de données (4) ;
- la source de tension électrique (3) est configurée pour alimenter, avec la tension électrique donnée, chaque photomultiplicateur en silicium (l) ;
- l'unité de commande est configurée pour choisir en séquence un élément optique (2) et pour définir une association optique et électrique entre l'élément optique (2) choisi et au moins un photomultiplicateur en silicium (1) et pour définir une valeur d'énergie électrique devant alimenter l'élément optique (2) choisi ;
- l'unité de commande numérique et d'élaboration de données (4) étant configurée pour alimenter, avec la valeur d'énergie électrique définie par l'unité de commande, l'élément optique (2) choisi et l'unité de mesure (5) étant configurée pour mesurer la valeur de l'état excité des photomultiplicateurs en silicium (1) de l'association ;
- ladite unité de commande étant configurée pour modifier la valeur de l'énergie électrique devant alimenter l'élément optique (2) choisi lorsque la valeur de l'état excité d'au moins un photomultiplicateur en silicium (1) de l'association est inférieure à la valeur linéaire minimale ou supérieure à la valeur linéaire maximale de l'état linéaire ;
- ladite unité de commande étant configurée pour stocker, dans l'unité de mémoire, la valeur d'énergie électrique devant alimenter l'élément optique (2) choisi lorsque la valeur de l'état excité de chaque photomultiplicateur en silicium (1) de l'association est comprise entre la valeur linéaire minimale et maximale de l'état linéaire.

5. Système selon une quelconque revendication 1 à 4, dans lequel les au moins deux sources d'émission peuvent être du même type ou d'un type différent.

6. Système selon une quelconque revendication 1 à 5, dans lequel l'une des deux longueurs d'onde moyennes de lumière émises doit être inférieure et l'autre supérieure à un point de longueur d'onde spécifique.

7. Procédé d'analyse de tissu cérébral comprenant les étapes suivantes :
- fournir un système selon une quelconque revendication 1 à 6 ;
- disposer les photomultiplicateurs en silicium (1) et les éléments optiques (2) sur la tête d'un patient selon un motif géométrique donné ;
- répéter en séquence les étapes suivantes jusqu'à ce que chaque photomultiplicateur en silicium (1) ait été associé à au moins un élément optique (2) et que chaque élément optique (2) ait été associé à au moins un photomultiplicateur en silicium (1) :
a) choisir, par l'unité de commande, un élément optique (2) ;
b) définir, par l'unité de commande, une association optique et électrique de l'élément optique (2) choisi et d'au moins un photomultiplicateur en silicium (l) ;
c) définir, par l'unité de commande, une valeur d'énergie électrique devant alimenter l'élément optique (2) choisi ;
d) alimenter, par l'unité de commande numérique et d'élaboration de données (4), avec la valeur d'énergie électrique définie par l'unité de commande, l'élément optique (2) choisi ;
e) mesurer, par l'unité de mesure (5), la valeur de l'état excité de chaque photomultiplicateur en silicium (1) de l'association et vérifier, par l'unité de commande, si la valeur de l'état excité de chaque photomultiplicateur en silicium (1) de l'association est comprise entre la valeur linéaire minimale et maximale de l'état linéaire ;
f) modifier, par l'unité de commande, la valeur d'énergie électrique devant alimenter l'élément optique (2) choisi si la valeur de l'état excité d'au moins un photomultiplicateur en silicium (1) de l'association n'est pas comprise entre la valeur linéaire minimale et maximale de l'état linéaire et répéter les étapes d) à f) ;
g) stocker, dans l'unité de mémoire, par l'unité de commande, la valeur d'énergie électrique devant alimenter l'élément optique (2) choisi conjointement avec l'association correspondante, si la valeur de l'état excité de chaque photomultiplicateur en silicium (1) de l'association est comprise entre la valeur linéaire minimale et maximale de l'état linéaire.

8. Procédé selon la revendication 7, dans lequel l'étape consistant à disposer les photomultiplicateurs en silicium (1) et les éléments optiques (2) sur la tête d'un patient selon un motif géométrique donné consiste à :
- disposer les photomultiplicateurs en silicium (1) et les éléments optiques (2) sur la tête d'un patient selon un motif en damier ; ou
- disposer les photomultiplicateurs en silicium (1) et les éléments optiques (2) sur la tête d'un patient selon un motif en nid d'abeilles.

9. Procédé selon la revendication 7 ou 8, dans lequel l'étape consistant à définir, par l'unité de commande, une association optique et électrique de l'élément optique (2) choisi et d'au moins un photomultiplicateur en silicium (1) consiste à :
- définir toutes les distances physiques entre l'élément optique (2) choisi et chaque photomultiplicateur en silicium (1) ;
- choisir, par l'unité de commande, une valeur de distances physiques parmi toutes les valeurs définies ;
- définir, par l'unité de commande, une association entre l'élément optique (2) choisi et la totalité des photomultiplicateurs en silicium (1) espacés de l'élément optique (2) choisi par la valeur de distance physique choisie à l'étape précédente ;
- stocker, par l'unité de commande, dans l'unité de mémoire, l'association définie à l'étape précédente conjointement avec la valeur correspondante de distance physique entre l'élément optique (2) et chaque photomultiplicateur en silicium (1) de l'association.

10. Procédé selon la revendication 9, dans lequel, après la répétition en séquence des étapes a) à g), le procédé consiste à :
- définir, par l'unité de commande, au moins un groupe d'associations, chaque association du même groupe étant **caractérisée par** la même distance physique entre l'élément optique (2) et chaque photomultiplicateur en silicium (1) de l'association ;
- stocker, dans l'unité de mémoire, par l'unité de commande, chaque groupe d'associations ;

11. Procédé selon la revendication 10, dans lequel le procédé comprend les étapes supplémentaires consistant à :
- activer en séquence chaque groupe d'associations par l'alimentation, par l'unité de commande numérique et d'élaboration de données (4), de chaque élément optique (2) de chaque association du même groupe avec la valeur correspondante d'énergie électrique définie par l'unité de commande ;
- mesurer, par l'unité de mesure (5), et stocker dans l'unité de mémoire, par l'unité de commande, après l'activation de chaque groupe d'associations, une valeur opérationnelle de l'état excité de chaque photomultiplicateur en silicium (1) de chaque association du même groupe ;
- retirer la totalité des éléments optiques (2) et des photomultiplicateurs en silicium (1) de la tête du patient ;
- fournir un corps de commande (20) ;
- disposer les éléments optiques (2) et les photomultiplicateurs en silicium (1) sur une surface supérieure (21) du corps de commande (20) selon le même motif géométrique que celui utilisé pour disposer les éléments optiques (2) et les photomultiplicateurs en silicium (1) sur la tête d'un patient ;
- activer en séquence chaque groupe d'associations par l'alimentation, par l'unité de commande numérique et d'élaboration de données (4), de chaque élément optique (2) de chaque association du même groupe avec la même valeur d'énergie électrique que celle alimentant les éléments optiques (2) lorsqu'ils sont positionnés sur la tête d'un patient ;
- mesurer, par l'unité de mesure (5), et stocker dans l'unité de mémoire, par l'unité de commande, après l'activation de chaque groupe d'associations, une valeur au repos de l'état excité de chaque photomultiplicateur en silicium (1) de chaque association du même groupe ;
- élaborer, par l'unité de commande, la valeur opérationnelle et la valeur au repos de l'état excité de chaque photomultiplicateur en silicium (1) de chaque association de chaque groupe d'associations pour définir une valeur mise à jour correspondante de l'état excité.

12. Procédé selon la revendication 11, dans lequel l'étape consistant à élaborer, par l'unité de commande, la valeur opérationnelle et la valeur au repos de l'état excité de chaque photomultiplicateur en silicium (1) de chaque association de chaque groupe d'associations pour définir une valeur mise à jour correspondante de l'état excité consiste à :
- définir, par l'unité de commande, la valeur mise à jour de l'état excité de chaque photomultiplicateur en silicium (1) de chaque association de chaque groupe d'associations comme étant la division entre la valeur opérationnelle correspondante de l'état excité et la valeur au repos correspondante de l'état excité ; ou
- définir, par l'unité de commande, une valeur mise à jour de l'état excité de chaque photomultiplicateur en silicium (1) de chaque association de chaque groupe d'associations comme étant la soustraction entre la valeur opérationnelle correspondante de l'état excité et la valeur au repos correspondante de l'état excité.
